# EUROPÄISCHE PATENTANMELDUNG

(11) **EP 1 518 524 A1**
(43) Veröffentlichungstag der Anmeldung: **30.03.2005**
(21) Anmeldenummer: 04017898.0
(22) Anmeldetag: 28.07.2004
(51) Int. Cl.: A61F 7/00, A61H 35/00

(54) **Verfahren und Vorrichtung zur äusserlichen Fluid-Behandlung eines menschlichen und/oder tierischen Körpers**

(30) Priorität: 28.07.2003 DE 10334349
(71) Anmelder: Gunnar Steins, 71263 Weil der Stadt (DE)
(72) Erfinder: Gunnar Steins, 71263 Weil der Stadt (DE)
(74) Vertreter: nospat Patentanwälte

(57) **Zusammenfassung**

Die vorliegende Erfindung betrifft ein Verfahren und eine Vorrichtung zur äußerlichen Fluid-Behandlung eines menschlichen und/oder tierischen Körpers (2), bei dem ein zu behandelnder Bereich der Körperoberfläche (6) mit einer undurchlässigen Schicht (3) abgedeckt und ein Fluid (5) unter dieser Schicht (3) eingeleitet so wird, dass es sich in Kontakt mit der Körperoberfläche (6) befindet.

Um eine Vorrichtung und ein Verfahren der vorstehend genannten Art dahingehend zu verbessern, dass bei vereinfachter Handhabung und weiter gemindertem Vorrichtungsaufwand die Menge eines je Badevorgang verbrauchten Fluids noch weiter gesenkt wird, wird vorgeschlagen, dass eine zusätzliche mindestens teilweise Fluid- durchlässige Schicht (4) zwischen der undurchlässigen Schicht (3) und der Körperoberfläche (6) angeordnet wird und das Fluid (5) derart zwischen diesen Schichten (3, 4) eingeleitet wird, dass die mindestens teilweise Fluid-durchlässige Schicht (4) an die Körperoberfläche (6) angelegt wird und eine geringe Menge an Fluid (5a) durch diese mindestens teilweise Fluid-durchlässige Schicht (4) hindurch auf einen Teil des abgedeckten Bereichs der Körperoberfläche (6) austritt.

## Beschreibung

Die vorliegende Erfindung betrifft ein Verfahren und eine Vorrichtung zur äußerlichen Fluid-Behandlung eines menschlichen und/oder tierischen Körpers.

Es sind vielfältige Formen derartiger Verfahren und Vorrichtungen bekannt, die bei Medizinern, Heilpraktikern und im Beauty- sowie Wellness-Bereich, aber auch in Tierarztpraxen und ähnlichen Einrichtungen eingesetzt werden. Ziel der jeweiligen Behandlung ist beispielsweise das Lösen muskulärer Verspannungen, die Linderung der Folgen rheumatischer Erkrankungen oder eine Behandlung von Reizungen und/oder Erkrankungen der Haut. In der Regel wird hierzu ein Fluid als Bad oder Spülung in Kontakt mit einem zu behandelnden Bereich der Körperoberfläche gebracht. Hierbei ist jedoch häufig eher eine Badevorrichtung, als nur ein Eimer oder ein Topf als Verabreichungsvorrichtung erforderlich.

Flexible Badevorrichtungen, die auch zu dem vorstehend genannten Zweck verwendet werden können, sind beispielsweise aus der CH 1 99 145 bekannt. Gemäß dieser Lehre besteigt eine Person zum Baden eine mit einem Fluid gefüllte Hülle, die daraufhin im Hals-Bereich durch Verschnüren geschlossen wird.

Zur Senkung des Bedarfs an Fluid gegenüber einem Vollbad schlägt die DE 43 42 135 A1 die Verwendung einer den jeweiligen Körper hautnah umgebenden Hülle vor. Die Hülle soll durch ein Bademedium vom Hals-Bereich bis zu den Füßen hin durchströmt werden.

Einen flexiblen Folienanzug aus einem wasserdichten Material schlägt auch die DE 195 31 856 A1 vor, wobei sich in der Hülle des Folienanzugs ein Bademedium befindet und eine zu badende Person sich mit diesem am Körper geschlossenen Anzug in eine mit einem weiteren Medium gefüllte Wanne legt. Die Folie des Anzugs wird durch den Druck des zweiten Mediums gleichmäßig gegen die Körperoberfläche gedrückt, so dass die gewählte Badeflüssigkeit im Wesentlichen die gesamte durch den Anzug abgedeckte Haut genetzt.

Es ist Aufgabe der vorliegenden Erfindung, ein Verfahren und eine Vorrichtung der vorstehend genannten Art dahingehend zu verbessern, dass bei vereinfachter Handhabung und weiter gemindertem Vorrichtungsaufwand die Menge eines je Badevorgang verbrauchten Fluids noch weiter gesenkt wird.

Diese Aufgabe wird erfindungsgemäß durch ein Verfahren und eine Vorrichtung mit den Merkmalen der unabhängigen Ansprüche gelöst. Vorteilhafte Weiterbildungen der Erfindung sind Gegenstand der jeweiligen Unteransprüche.

Der Erfindung liegt die Erkenntnis zugrunde, dass für eine Behandlung eines Körpers ein Kontakt zwischen einem ausgewählten Fluid und der Haut entscheidend ist. Als weitgehend unerheblich stellt sich die Mächtigkeit einer jeweiligen Fluidschicht heraus. Damit wurde erfindungsgemäß eine dünne und nur für eine im Wesentlichen durchgehende Benetzung der zu behandelnden Hautoberfläche ausreichende Fluidschicht ausgewählt. Ein derartiges Verfahren ist gegenüber bekannten Verfahren auch sehr sparsam, da zum Aufbau einer derartigen Schicht auch nur eine sehr geringe Menge an Fluid erforderlich ist.

Das Problem einer gleichmäßigen Verteilung einer nur sehr geringen Fluidmenge wird erfindungsgemäß dadurch gelöst, dass eine zusätzliche, in Randbereichen mit der undurchlässigen Schicht verbundene, mindestens teilweise Fluid-durchlässige Schicht zwischen der undurchlässigen Schicht und dem Körper bzw. der Hautoberfläche angeordnet wird. Das Fluid wird über einen Zulauf derart zwischen diesen Schichten eingeleitet, dass die mindestens teilweise Fluid-durchlässige Schicht an den Körper angelegt wird und eine geringe Menge an Fluid durch Öffnungen dieser mindestens teilweise Fluid- durchlässigen Schicht hindurch auf einen Teil des abgedeckten Bereichs der Körperoberfläche austritt. Hierzu sind in der mindestens teilweise Fluid-durchlässigen Schicht vorzugsweise Mikro-Öffnungen zum Fluid-Austritt vorgesehen.

Eine dementsprechende Vorrichtung umfasst demnach eine Fluid-undurchlässige Schicht und eine mindestens teilweise Fluid-durchlässige Schicht, die an freien Endbereichen miteinander derart verbunden sind, dass zwischen ihnen ein Fluid eingefüllt werden kann. Aus der gewählten Anordnung ergibt sich unmittelbar der vorteilhafte Effekt, wonach sich beim Füllen einer vorzugsweise in Randbereichen geschlossenen zweilagigen Struktur, die einen zu behandelnden Bereich der Körperoberfläche abdeckend fixiert ist, eine zum Körper orientiert angeordnete und mindestens teilweise Fluid-durchlässige Schicht an die Körperoberfläche andrückt.

Durch das Andrücken der mindestens teilweise Fluid- durchlässigen Schicht an die Körperoberfläche wird automatisch neben einem optimierten Sitz einer erfindungsgemäßen Vorrichtung auch eine Art von Abdichtung um jede Fluid-Austrittsöffnung herum bewirkt, so dass im Gegensatz zu Vorrichtungen nach dem Stand der Technik ein Auslaufen des Fluids auch ohne weitere Dichtungsmaßnahmen nicht vorkommen kann. Damit wird ein erfindungsgemäßer Aufbau vereinfacht. Zudem wird die Handhabung einer derartigen Vorrichtung ganz wesentlich erleichtert: Eine Grundbauform einer erfindungsgemäßen Vorrichtung wird in Form eines entleerten Kissens auf einem zu behandelnden Bereich der Körperoberfläche fixiert, beispielsweise durch eine Bandagierung oder Verschnürung. Beim Einfüllen von Fluid dehnt sich die Vorrichtung aus und bewirkt dabei automatisch auch bei vormals lockerer Fixierung der Vorrichtung eine ausreichende Lagesicherung. Spätestens mit Erreichen eines anwendungsspezifischen Drucks des Fluids in der Vorrichtung tritt der vorteilhafte Effekt der Selbstabdichtung auf.

In einer Weiterbildung der Erfindung wird über oder um eine zu behandelnde Region eine manschettenartige Vorrichtung umschließend angeordnet. Das Fluid wird wiederum in diese Vorrichtung bei einem bestimmten Druck eingeleitet, so dass die Vorrichtung an die Haut angelegt wird. Das Fluid wird durch einen Fluid-durchlässigen Bereich der Vorrichtung auf die Haut in definierter Weise ausgelassen, wobei umliegende Bereiche der Vorrichtung durch ihre Anlage an der Haut eine Abdichtung bewirken. Durch das Material des mindestens einen Fluid-durchlässigen Bereichs der Vorrichtung, aber auch den angewendeten Druck auf das Fluid, ist der Austrag an Fluid auf die Haut regulierbar. Während eine Verabreichung und genaue Dosierung von Medikamenten, Heilmitteln oder kosmetisch wirksamen Substanzen etc. nach dem Stand der Technik sehr problematisch sein kann, erfolgt sie erfindungsgemäß in einfach einstellbarer Weise über die vorstehenden Regelungsparameter und insbesondere für die Öffnungsdichte und den damit verbundenen Fluid-Austrag auf eine durch die Über- oder Abdeckung festgelegten Bereich einer Körperoberfläche.

Eine Vorrichtung ist also erfindungsgemäß dadurch gekennzeichnet, dass sie einen Fluid-undurchlässigen Außenbereich und einen Fluid-durchlässigen Innenbereich aufweist, der bei erfindungsgemäßem Gebrauch einer zu behandelnden Region zugewandt angeordnet ist. Die Vorrichtung ist zur Befüllung mit einem Fluid unter einem Innendruck ausgebildet, der vorzugsweise in einem Bereich von 0,01 bar bis unter 1 bar, insbesondere aber von ca. 0,2 bis 0,8 bar liegt.

Bei Vorrichtungen nach dem Stand der Technik treten neben dem Aufwand, einen in einer Wanne zu tragenden Anzug bereitzustellen, z.B. bei Umsetzung der Lehre der DE 195 31 856 A1 und den vorstehend diskutierten Druckschriften das Problem einer Anpassung an unterschiedliche Größen einer zu behandelnden Person sowie die Notwendigkeit mindestens einer Abdichtung auf. Unter Einwirkung eines Fluids schmiegt sich eine erfindungemäße Vorrichtung hingegen mit der mindestens teilweise Fluid- durchlässigen Schicht im Wesentlichen durchgängig und flächendeckend an eine zu behandelnde Körperregion an, so dass sie einerseits eine Größenanpassung der Vorrichtung und deren Lage-Fixierung bewirkt, andererseits jedoch in einstellbarer Dosierung das Fluid in geringer Menge auf die zu behandelnde Körperoberfläche abgibt. Durch das Fluid erfolgt also in einer erfindungsgemäßen Vorrichtung quasi ein Aufpumpen einer doppelwandigen Anordnung, so dass sich neben einer Abdichtung durch Anlegen des Innenbereichs an der Körperoberfläche automatisch auch eine Größenanpassung über einen sehr weiten Bereich hinweg neben einer Abdichtung ergibt.

Es wird erfindungsgemäß also eine doppelwandige Anordnung vorgesehen, die als Kompresse, Manschette oder größere Einheit ausgebildet sein kann und sich unter eigenem Innendruck bzw. Innendruck des Fluids selber an die Haut einer zu behandelnden Person andrückt. Dabei wird durch Poren oder ähnliche Mikro-Öffnungen wählbarer Lage und Dichte nur ein sehr geringer Teil des Fluids im Wesentlichen nur befeuchtend auf eine jeweils abgedeckte Hautpartie ausgetragen. Das restliche Fluid gerät mit einer zu behandelnden Person vom Prinzip her nicht in Kontakt, so dass es als unberührtes oder unkontaminiertes Fluid ohne jegliche Aufbereitung bei einem weiteren Patienten eingesetzt werden kann. Damit ist ein Verbrauch an Fluid durch Kontamination über einen Hautkontakt mit der zu behandelnden Person gegenüber allen derzeit bekannten Vorrichtungen und Verfahren nochmals deutlich gesenkt worden.

Bei der vorstehend beschriebenen Lösung kann eine Temperierung gerade eines während einer Anwendung unter Druckbeaufschlagung ruhenden Fluids durch Wärmestrahlung von außen her oder durch eine Heizfolie als Bestandteil der Vorrichtung vorgenommen werden. In einer wesentlichen Weiterbildung weist eine erfindungsgemäße Vorrichtung jedoch neben einer Füllöffnung und einem Entlüftungsventil auch einen Abfluss auf, so dass die vorstehend beschriebene Vorrichtung durch das Fluid im Wesentlichen frei durchströmt werden kann. Daher kann das Fluid auch die Aufgabe einer Temperierung als Zusatzfunktion übernehmen, indem eine Temperatureinstellung über einen Zuund Abstrom von Fluid und dessen Zuflusstemperatur eingeregelt wird. Ferner bietet bereits dieser einfache Basis-Aufbau durch pulsende Beaufschlagung mit fließendem Fluid eine sehr effektive Möglichkeit zur Durchführung von aktiven mechanischen Massagen. Bereits nach dem Stand der Technik werden bei Fluid-Kontakten der Haut auch Temperaturbehandlungen und Behandlungen in Kombination mit sonstiger elektromagnetischer Strahlung vorgenommen, was in einer erfindungsgemäßen Vorrichtung in intensivierter Weise realisierbar ist.

In dem Fachmann naheliegender Weise können derartige bekannte Maßnahmen auch in einer erfindungsgemäßen Vorrichtung integriert werden. Während aber für eine mechanische Unterstützung einer Behandlung nach dem Stand der Technik in der Regel zusätzliche Gerätschaften benötigt werden, so kann durch eine Variation der Druckbeaufschlagung und/oder eine interne Einteilung in mehr als einen Versorgungskreis eine mechanische Einwirkung ebenfalls über das Fluid erreicht werden.

In einer bevorzugten Ausführungsform der Erfindung wird die nach den vorstehenden Ausführungen zwei Schichten umfassende Abdeckung über den Zulauf durch eine Versorgungseinheit mit Fluid versorgt. Das Fluid wird dabei in der Versorgungseinheit hinsichtlich Temperatur, Druck, Pulsung, Zusammensetzung und/oder Beimischungen regelbar und/oder einstellbar sowie in bakteriologischer und hygienischer Hinsicht durch Filterung, Bestrahlung, oligodynamische und/oder thermische Prozesse u.a. aufbereitet. Entsprechende Mittel werden nachfolgend unter Bezugnahme auf die Zeichnung mit der Darstellung von Ausführungsbeispielen noch im Detail beschrieben.

Die vorstehend genannten Aufgaben werden also in erfindungsgemäßen Ausführungsformen zusammengefasst, so dass neben einem Einsatz an einem professionellen Einsatzort mit höchsten Hygieneansprüchen auch Behandlungen und im Wesentlichen Entspannungen auch im heimischen bzw. privaten Bereich für eine oder wenige Einzelpersonen unter Minderung bakteriologischer und hygienischer Aspekte durchgeführt werden können.

Anwendung findet eine derartige Vorrichtung u.a.:
1. im medizinischen Bereich, wo insbesondere in Wasser gelöste Salze, Mineralien, ätherische Öle, hautheilende Substanzen und/oder Medikamente auch unter Sauerstoff- oder sonstiger Gase und Mischgaszusetzung zur Behandlung von Hautkrankheiten benutzt werden, wobei durch eine erfindungsgemäße Vorrichtung neben einer direkten Behandlung eines vorbestimmten Hautbereiches auch eine Vorbereitung der Haut vor eine nachfolgende Cremung möglich ist. So ist insgesamt eine Tiefenwirkung bis in die Lederhaut-Schicht hinein erreichbar;
2. im homöopathischen Bereich: Hier können bei Rücken-, Wirbelsäulenschmerzen oder Muskelverzerrungen durch Zugabe von Rheumamitteln, stärkerer Pulsung und einer weichen Einstellung im Liegekomfort große Erleichterungen und/oder Schmerzlinderung erzielt werden;
3. im Kurbereich, wenn durch warmes Wasser, das mit Extrakten durchsetzt ist, einer sanften Pulsung und dem Liegekomfort, wie auf einem Luftkissen, sowie unter gezieltem Temperatureinfluss relaxe Zustände erreicht werden. Unter Verwendung von Heilwässern, Solen etc. kann eine erhebliche Minderung des derzeitigen Aufwandes bei einem Einsatz auch für gebrechliche Menschen erzielt werden;
4. im Bereich von Wellness und Schönheitspflege, wenn dem Wasser z.B. hautgeschmeidigmachende Flüssigkeiten zugeführt werden, eine höhere Wärmeeinstellung Arterienöffnungen bewirkt und mit entsprechender Pulsung eine gute Durchblutung des Körpers erreicht wird;
5. im privaten Bereich, da hier unproblematisch, wann auch immer Zeit ist, eine verhältnismäßig schnelle und tiefe Entspannungsphase durch entsprechende Einstellungen der Wasserwärme, dem Liegekomfort und der Pulsung erreicht werden kann und
6. im Rettungsbereich, wo nun Unterkühlungen von Personen, die erst nach längerer Zeit geborgen wurden, direkt vor Ort und auch während eines Transportes behandelt werden können. Umgekehrt sind aber selbstverständlich auch Verbrennungsverletzungen mit einer erfindungsgemäßen Vorrichtung behandelbar.

Nachfolgend werden Ausführungsbeispiele der Erfindung anhand der Zeichnung zur Darstellung weiterer Merkmale und Vorteile näher erläutert. Es zeigt
- Figur 1:: eine Schnittdarstellung durch einen erfindungsgemäßen Aufbau einer Vorrichtung im Einsatz;
- Figur 2a und 2b:: eine Draufsicht auf abgewandelte Ausführungsformen einer ersten Vorrichtung;
- Figur 3:: eine Draufsicht auf eine weitere Ausführungsform einer Vorrichtung;
- Figur 4:: eine Draufsicht auf eine weitere angepasste Ausführungsform einer Vorrichtung;
- Figur 5:: eine Schnittdarstellung einer weiteren Ausführungsform;
- Figur 6:: eine Darstellung einer weiteren Ausführungsform der Erfindung mit einer Versorgungseinheit;
- Figur 7:: eine Reihendarstellung von Applikatoren gemäß der Figuren 2a bis 6;
- Figur 8:: eine Prinzipdarstellung einer weiteren Versorgungseinheit und
- Figur 9:: eine Darstellung einer weiteren Versorgungseinheit.

Nachfolgend werden in den unterschiedlichen Ausführungsbeispielen gleiche Bestandteile zur Erleichterung der Orientierung durchgängig mit den gleichen Bezugszeichen bezeichnet werden.

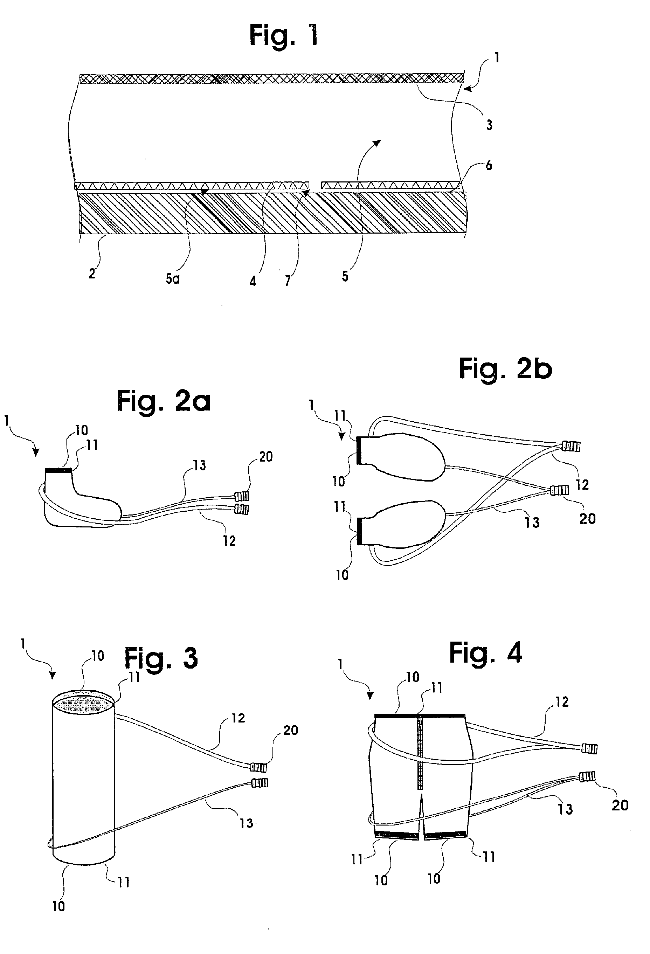

Eine erfindungsgemäße Vorrichtung 1 zur äußerlichen Fluid-Behandlung eines menschlichen Körpers 2 umfasst gemäß Figur 1 eine Fluid-undurchlässige Schicht 3 und eine mindestens teilweise Fluid-durchlässige Schicht 4. Beide Schichten 3, 4 decken einen zu behandelnden Bereich der Körperoberfläche ab und zwischen diesen Schichten 3, 4 wird ein Fluid 5 derart eingeleitet, dass die mindestens teilweise Fluid-durchlässige Schicht 4 an den Körper 2 angelegt wird.

Aufgrund der Tatsache, dass die Schicht 4 mindestens teilweise Fluid-durchlässig ist, tritt eine geringe Menge an Fluid 5 durch diese Schicht 4 hindurch auf den von der Vorrichtung 1 abgedeckten Bereich des Körpers 2 aus. Da die teilweise Fluid-durchlässige Schicht 4 andererseits jedoch durch den Fluid-Innendruck an die Körperoberfläche 6 angedrückt wird, kann das ausgetretene Fluid 5a nicht vom Körper 2 ablaufen. Das über Mikro-Öffnungen 7 ausgetretene Fluid 5a verweilt für die Dauer einer Druckbeaufschlagung an der Körperoberfläche 6, so dass über eine definierte Zeitspanne hinweg in dem Fluid 5 enthaltene Wirkstoffe über die Körperoberfläche 6 auf diese und den Körper als ganzen einwirken können.

In einer nicht weiter dargestellten Grundform ist die Vorrichtung 1 als im Wesentlichen kissenförmige Kompresse ausgebildet. Dazu sind die Fluid-undurchlässige Schicht 3 als faserverstärkte, flexible Kunststofffolie und die mindestens teilweise Fluid-durchlässige Schicht 4 als sog. Pinaker-Folie oder Ripstopp-Nylon ausgebildet. Beide Materialien sind reißfest, keimresistent und wasserdurchlässig. Beide Schichten 3, 4 werden unter Ausbildung einer Fluid-Zufuhröffnung in einem thermischen Schneid-Klebeverfahren an ihren im Wesentlichen deckungsgleichen Außenrändern Fluid-dicht miteinander verbunden.

Das Anlegen der beschriebenen Vorrichtung 1 läuft dabei denkbar einfach ab. So kann bereits eine lockere Fixierung einer derartigen Vorrichtung 1 an einem Körper 2 zur Lagesicherung auch während einer Behandlung ausreichen, beispielsweise durch eine elastische Bandage oder eine Verschnürung. Bei Einleitung des Fluids 5 in die Vorrichtung 1 vergrößert sich deren Volumen und spannt eine zuvor auch nur locker angebrachte Bandage. So bildet sich ab einer bestimmten Füllmenge an Fluid 5 in der Vorrichtung 1 ein Anlagedruck zwischen der teilweise Fluid-durchlässigen Schicht 4 und der Körperoberfläche 6 aus, die ein weiteres Austreten von Fluid verhindert. In diesem Zustand ist die von der Vorrichtung 1 abgedeckte Körperoberfläche 6 im Wesentlichen unterbrechungsfrei und gleichmäßig über die abgedeckte Körperoberfläche 6 durch eine dünne Schicht 8 von Fluid 5a benetzt.

Das gleiche Wirkprinzip baut sich bei den in den Figuren 2a, 2b bis 4 als Prinzipskizzen dargestellten Ausführungsformen auf. Die voranstehend beschriebene im Wesentlichen ebene Struktur der Vorrichtung 1 ist hierbei in Anpassung auf Teile des menschlichen Körpers als dreidimensionale Struktur mit einer, zwei und drei Öffnungen 10 ausgebildet. So wird als Figur 2a ein Schuh zur Behandlung eines Fußes und in Figur 2b eine zu Handschuhen leicht abgewandelte Vorrichtung 1 zur simultanen Behandlung von zwei Händen vorgesehen. Eine Arm-, Bein- oder Torso-Manschette ist in Figur 3 skizziert. Figur 4 zeigt schließlich eine Vorrichtung 1, die nach Art einer Hose zur Behandlung eines erweiterten Beckenraumes ausgebildet ist. Eine jede Öffnung 10 ist in der angedeuteten Weise durch eine Ringwulst 11 umfasst, die aus dem Material der Fluid-undurchlässigen Schicht 3 besteht und bei Beaufschlagung der Vorrichtung 1 mit Fluid 5 ebenfalls oder zusätzlich als Abdichtung an die Körperoberfläche 6 angelegt wird.

Als Erweiterung gegenüber der vorstehend beschriebenen Grundform einer Vorrichtung 1 weisen die Beispiele der Figuren 2a bis 4 jedoch neben einem Zulauf 12 auch einen Ablauf 13 auf. Diese Vorrichtungen 1 können also gemäß der voranstehenden Beschreibung über den Zulauf 12 und ein Verschließen des Ablaufes 13 mit im Wesentlichen stationärer Befüllung durch das Fluid 5 verwendet werden. Hierbei wird vorzugsweise eine Temperierung während des Betriebs durch Wärmestrahlung von außen her oder durch eine körpernah angeordnete Heizfolie vorgenommen, die insbesondere ein Bestandteil der aus den beiden Schichten bestehenden Abdeckung ist.

Ferner ist es bei der vorstehend beschriebenen Ausführungsform der Erfindung jedoch auch möglich, einen Durchfluss von Fluid 5 durch eine jeweilige Vorrichtung 1 von einem Zulauf 12 zu einem Ablauf 13 hin auszubilden. Dieser Durchfluss an temperiertem Fluid 5 kann dann vorzugsweise zur Regulierung einer Temperatur in der Abdeckung bzw. der Vorrichtung 1 genutzt werden. Durch diese Maßnahme wird ein Auskühlen einer zu behandelnden Person in einfacher Weise verhindert. Andererseits wird ein Wohlbefinden durch eine regelbare Temperatur in der Vorrichtung ganz wesentlich gesteigert.

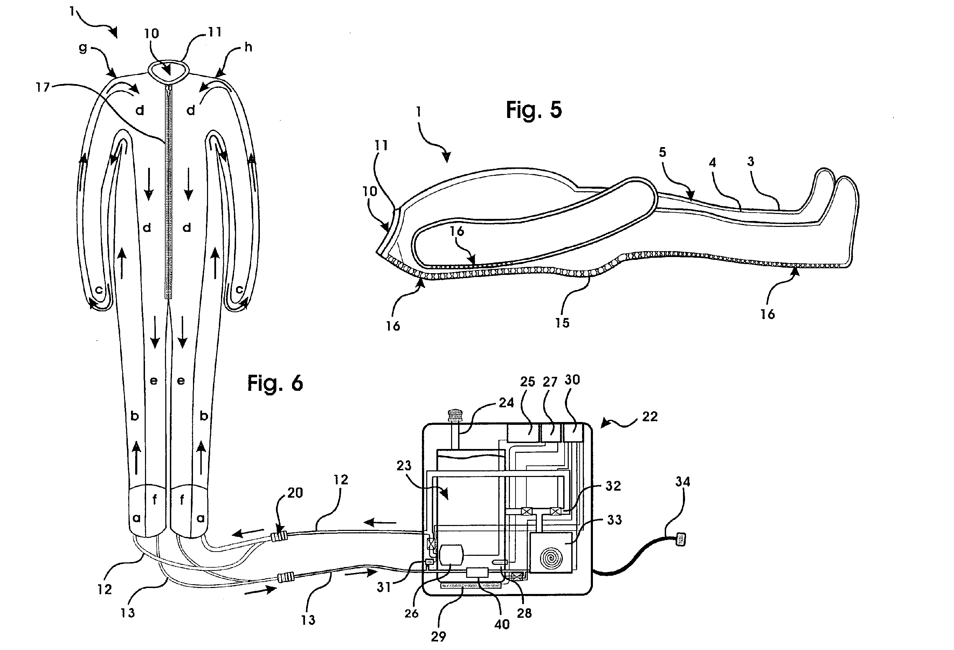

Für die Temperierung ferner wesentlich ist, dass sich keine sog. Kälte-Nester innerhalb der Vorrichtung 1 ausbilden können. Diese ist bei einer Fluid-Durchströmung einer großflächigeren Abdeckung des menschlichen Körpers 2 immer dort der Fall, wo beispielsweise durch die normalen Schwerkrafteinflüsse die Schichten 3, 4 soweit aufeinander gedrückt werden, dass diese Bereiche nicht länger effektiv mit temperiertem, neuem Fluid 5 als Austausch durchströmt werden können. Als Abhilfemaßnahme sind in einer skizzierten Schnittdarstellung nach Figur 5 in einer als Ganzkörperanzug ausgebildeten Vorrichtung 1 Stützkörper 15 zwischen den Schichten 3, 4 vorgesehen. Die Stützkörper 15 sind als ungefähr I-förmige Körper aus einem elastischen Material ausgebildet und bilden eine Art von Stützpolster 16. Statt einzelne Stützkörper 15 auszubilden und einzusetzen kann diese Stützfunktion durch einen offenporigen und damit gut durchströmbaren Schaumstoff in Form eines einstückigen Stützpolsters 16 realisiert werden. Hier bilden die Zellenwände des Stützpolsters 16 ebenfalls eine Art von Stützkörpern 15 mit der vorstehend beschriebenen Funktion und gleichen in einer Schnittdarstellung einer entsprechenden Anordnung von einzelnen Stützkörpern 15. Da eine Anwendung einer derartigen Vorrichtung 1 i.d.R. im Liegen erfolgen wird, ist ein Bereich der Vorrichtung 1, der einer Rückpartie mit Gesäß und Beinen mit normalem Kontakt mit einer Unterlage entspricht, in der nur angedeutet dargestellten Weise mit Stützpolstern 16 versehen. Auch Teilbereiche der Ärmel werden so ausgerüstet, um die Ausbildung von Stauungen und/oder strömungstoten Bereichen zu unterbinden. Die Stützpolster 16 werden ferner benötigt, damit ein liegender Patient weich liegt und genügend Wasser im Rückenbereich zirkulieren kann, da hier die Schwerkraft die innere und äußere Folie 3, 4 zusammenpresst. Dem wäre nur mit einem höheren Innendruck des Fluids 5 zu entgegnen, der aber wiederum einen zu starken Druck auf den Körper 2 ausüben würde. Durch Einbau der Stützpolster 16 kann mit einem relativ niedrigen Wasserdruck gefahren werden und der Patient hat nach eigener Wahl die Möglichkeit, die Vorrichtung straffer oder weniger straff anliegend einzustellen.

Ein weiteres Ausführungsbeispiel ist in Figur 6 skizziert. Hier ist die Vorrichtung 1 mit dem in Figur 1 dargestellten Grundaufbau als doppelwandiger Ganzkörperanzug ausgebildet, der den Körper 2 mit Füßen und Händen bis zum Hals umschließt. Die Vorrichtung 1 ist mit einem Reißverschluss 17 vom Hals bis zur Körpermitte versehen, damit er leicht und prinzipiell ohne weitere Hilfe aus- und anziehbar ist. Tatsächlich und in zeichnerisch nur schwer wiedergebbarer Weise ist die Vorrichtung 1 als doppelwandiger Ganzkörperanzug in ein- oder mehrteiliger Ausgestaltung ausgebildet, der einen Körper 2 mit Füßen und Händen bis zum Hals umschließt, jedoch den Bereich der Achseln und/oder der Genitalien aus hygienischen Gründen ausspart.

Der Anzug 1 umfasst eine innere, leicht perforierte, hautähnliche und in Berührungskontakt mit der Körperoberfläche 6 stehende teilweise Fluid-durchlässige Schicht 4. Diese teilweise Fluid-durchlässige Schicht 4 ist als in sich unterteilte Folie ausgebildet und mit einer äußeren und Fluid- undurchlässigen Schicht 3 in Form einer Gewebefolie überdeckt. In Randbereichen zusammenverschweisst bildet diese Anordnung 1 in diesem Beispiel mehrere miteinander verbundene Kammern als System mit vorgegebenen Wasserkreisläufen. An den unteren Anzugenden 18, 19 sind zwei Zulauf- und Rücklaufschläuche 12, 13 mit Schnellkupplungen 20 angebracht, über die der Anzug 1 im Verbund einer Versorgungseinheit 22 mit dem Fluid 5 in Form von Wasser oder medizinischen Lösungen beschickt wird. Der Anzug 1 weist in diesem Ausführungsbeispiel nach Figur 6 ein verzweigtes Kammersystem auf, indem ein Vorlauf mit den Bereichen a, b, c und ein Rücklauf mit den Bereichen d, e, f voneinander getrennt sind. Ferner sind auch eine linke Anzugseite g und eine rechte Anzugseite h voneinander getrennt, mit dem Reißverschluss 17 als einem Teil einer Trennlinie. Das warme Fluid 5 wird jeweils von einem Vorlauf des Anzuges 1 über Zulaufschläuche 12 in die Fußbereiche a eingeleitet und über die Beinbereiche b, durch die Arme c, in die Rückenund Brustbereiche d geführt. Die schon leicht abgekühlte Flüssigkeit wird dann über die Beinbereiche e, und Fußbereiche f durch Rücklaufschläuche 13 aus dem Anzug 1 wieder abgezogen.

Ein System miteinander verbundener Kammern mit separat vorgegebenen Wasserkreisläufen wird in einer weiteren Ausführungsform zu einem mechanischen Massagesystem nach Art eines Lymphmassage-Systems ausgebildet. Dieses System kann mit stationärem oder fließendem Fluid 5 arbeiten und bewirkt neben einer Massage durch rhythmisch mit Druckbeaufschlagung ausgelöste Kontraktionen aufeinanderfolgender Bereiche in der Vorrichtung 1 selbstverständlich weiterhin und ungestört parallel laufend eine Fluidbehandlung der abgedeckten Körperoberfläche 6.

Hier wurden statt des Einsatzes einer Drossel im Ablaufbereich Durchmesserunterschiede in den Zulauf- und Rücklaufschläuchen 12, 13 zum Druckaufbau gewählt. Ein einzustellender Innendruck wird über die Pumpleistung reguliert. Wie die Einstellung eines Drucks wird auch eine Temperierung und Pulsung des Fluids 5 in einer Versorgungseinheit 22 vorgenommen. Die Versorgungseinheit 22 umfasst einen Druckbehälter 23 mit Einfüllstutzen 24, eine Frequenzeinheit 25 mit Pulser 26, eine Temperaturregeleinheit 27 mit Temperaturfühler 28 und Aufwärmeinheit 29, eine Druckregeleinheit 30 mit Drucksensor 31, Ventile 32 und Druckpumpe 33. Ferner ist ein nicht weiter dargestellter DVD-Spieler integriert, dessen Aufzeichnungen über eine separate Beschallungsanlage zum Ohr und/oder über den Pulser 26 und das Fluid 5 der Vorrichtung 1 zugeführt werden. Auch können auf diesem Medium Steuerungsinformationen in Form eines vorgegebenen Behandlungsprogramms enthalten sein. Dabei ist in an sich gewohnter Weise auch ein Anschluss an einen Rechner als zentrale Kontroll- und Regelungseinheit mit Zahlungsfunktion, Statik und Wartungsintervall-Kontrolle etc. vorgesehen.

In der Versorgungseinheit 22 sitzt gemäß einer bevorzugten Ausführungsform der Erfindung eine Absaugpumpe 40, die mit dem Ablaufschlauch 13 und mit der Druckpumpe 33 verbunden ist. Somit können die Flüssigkeiten an den Anwendungsteilen und Applikatoren der unter Bezug auf die Abbildungen der Figuren 2a bis 6 dargestellten Formen nach der Behandlung automatisch abgesaugt werden. Dies erleichtert das Aussteigen des Patienten aus jeder der erfindungsgemäßen Vorrichtungen 1.

Der Druckbehälter 23 dient auch als Ausgleichs- und Vorratsbehälter für das Fluid 5. Der Verbrauch an Fluid 5 ist durch die geringe jeweils pro Behandlung oder Sitzung austretende Menge an Fluid 5a insgesamt sehr niedrig. Da im Wesentlichen kein Fluid 5a nach Kontakt mit der Körperoberfläche 6 mehr durch die mindestens teilweise Fluid-durchlässige Schicht 4 wieder in umgekehrter Richtung hindurch tritt, kann das im Druckbehälter 23 enthaltene Fluid 5 ohne Aufbereitung oder Reinigung sofort für die nächste Behandlung eingesetzt werden.

Die in der Gesamtansicht gezeigte Anlage aus Anzug 1 und Versorgungseinheit 22 wird über einen Versorgungsanschluss 34 mit Wechselstrom 220V betrieben. Sie kann aber auch mit entsprechende Modifikationen z.B. über eine 12-Volt Spannung autark an einer Batterie oder Solaranlage betrieben werden.

Unter Nutzung einer in der Regel bereits vorhandenen Infrastruktur kann die Vorrichtung 1 auch an einem normalen Wasserhahn angeschlossen werden. Eine Temperatureinstellung wird dann über die Armatur vorgenommen, in der im Fall einer Brause- oder Duscharmatur ein Thermostat enthalten sein kann. Eine derartige, hier nicht weiter dargestellte Vorrichtung ist mit einer integrierten Umwälzeinheit versehen und weist vorzugsweise auch ein Mehrwegeventil auf, um direkt am Anschluss im Bereich der Armatur ein Ablassen des Fluids 5 nicht der Behandlung zu ermöglichen. Ferner ist eine Vorrichtung zur Dosierung eines Wirkstoffs oder Medikaments vorgesehen, die auch zur Einspeisung eines Gases oder Gas-Einmischung in ein Fluid geeignet ausgebildet werden kann. Dann können auch kontrollierte Sauerstoff-, Ozon- oder Kohlendioxid-Behandlungen in ungefährlicher Weise im privaten Bereich vorgenommen werden.

Zur Säuberung der Anlage ist der Rücklaufschlauch 13 per Schnellkupplung 20 zwischen Versorgungseinheit 22 und dem Anzug 1 zu lösen. Das Schlauchende zur Versorgungseinheit 22 wird an einen Wasserhahn angeschlossen und Wasser oder einem extra Spülmittel, gegebenenfalls unter Zusatz eines Desinfektionsmittels, durch das System laufen gelassen. Das Wasser tritt dann am gelösten Rücklaufschlauch 13 des Anzuges 1 aus. Zusätzlich können Ablassventile an der Vorrichtung im Bereich der Extremitäten angeordnet sein, insbesondere an den Füßen des Anzugs 1. Dadurch wird der Ablauf von Fluid 5 und/oder Spülmittel beschleunigt. Durch selbsttätig schließende Schnellkupplung 20 auf beiden Seiten des System kann ein Nutzer den Anzug 1 von der Versorgungseinheit 22 trennen und jedes Teil für sich transportieren und versorgen.

Weiterhin kann das Innere des Anzuges 1 durch Öffnen des Reißverschlusses 17 mit Wasser und Seife gereinigt werden. Dazu wird der Anzug 1 auf links gezogen, so dass das Innere nach außen gekehrt wird. Der Anzug 1 kann verkehrt herum zum Trocknen aufgehängt oder weiter nach Art eines Tauchanzugs zur Reinigung und Trocknung behandelt werden. Auch eine Reinigung und Sterilisation in einem Autoklaven unter Gaseintrag ist möglich.

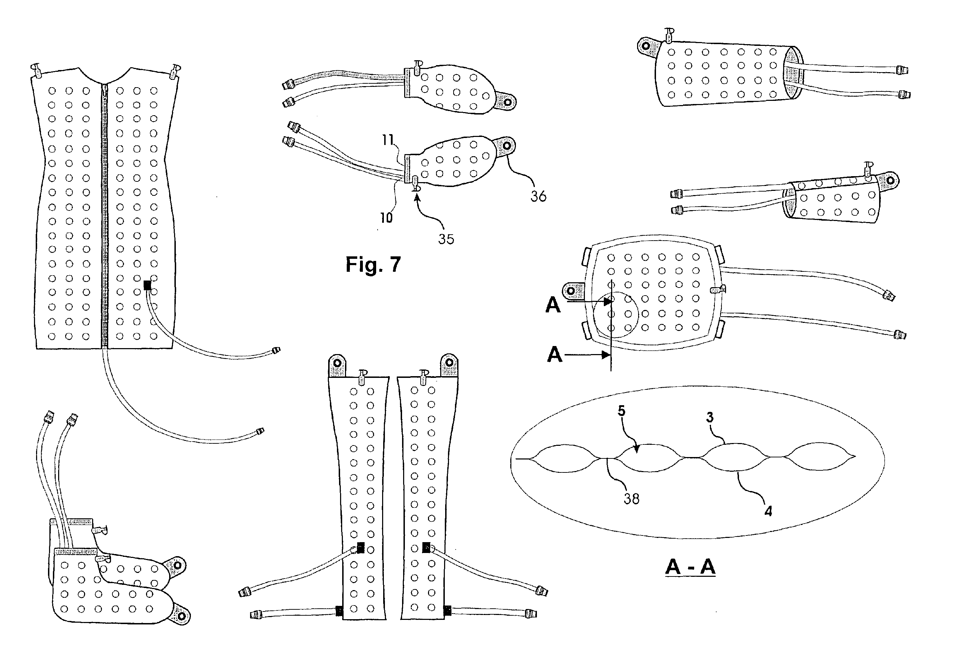

In einer weiteren Ausführungsform der Erfindung gemäß der Abbildungsfolge der Figur 7 wird einer Ausbildung von Kältenestern, also abkühlenden oder gar schon abgekühlten Ansammlungen von Fluid innerhalb eines Applikators bzw. Anwendungsteils, dadurch entgegengewirkt, dass die Fluid-durchlässige Schicht 4 mit der äußeren und Fluid-undurchlässigen Schicht 3 in der Fläche durch kreis- oder nahezu punktförmige Verbindungen 38 nach Art einer Steppdecke verbunden ist, wie diese in der Schnitt-Vergrößerung A-A in Figur 7 skizziert ist. Diese nahezu punktförmigen Verbindungen werden vorzugsweise in der Form von kreisförmigen Abschweißungen der Folien 3, 4 realisiert, die bei einem Durchmesser von ca. 20 mm einen Abstand zueinander von ungefähr 55 mm aufweisen. Sie sind über die Vorrichtung 1 regelmäßig verteilt, wobei Abweichungen u.a. zur Erhöhung der Beweglichkeit und Bequemlichkeit vorgesehen sein können. Durch die vorstehend angegebene Anbindung der Fluid-durchlässigen Schicht 4 an die Fluid-undurchlässige Schicht 3 wird eine Ausbildung von schwach durchströmten Fluid-Säcken auch gerade bei großflächigen Applikatoren verhindert, wie beispielsweise eines Hosenteils oder einer jackenförmigen Oberkörperabdeckung.

Neben den in der Zeichnung in der Form von Schnellkupplungen 20 für Zulauf- und Ablaufschläuche 12, 13 dargestellten Anschlüssen ist in dargestellten Ausführungsformen der Figur 7 gegenüber den Darstellungen der Figuren 2a bis 6 zusätzlich mindestens ein Entlüftungsventil 35 je Anwendungsteil vorgesehen. Hierdurch wird das Be- und Entfüllen erleichtert. Zudem sind je Anwendungsteil mindestens ein Aufhänglasche 36 an Endbereichen der Applikatoren angeformt. Am Beispiel der modifizierten Handschuhe von Figur 2b soll an dieser Stelle auch noch auf den konkreten Aufbau eingegangen werden: Die Folien 3, 4 bestehen beide aus dem gleichen 0,5 mm starken flexiblen PVC-Material, wobei die teilweise Fluid- durchlässige Innenfolie 4 mit einer durch Nadelkissen, Nadelrollen oder Laserung bewirkten Mikro-Perforierung versehen ist. Zum Randbereich der Öffnung 10 hin weist die Innenfolie 4 keine Perforierung mehr auf, auch erfolgt die Steppung mit den punktförmigen Verbindungen 38 in vergrößertem Abstand. So ergibt sich ca. 50 bis 70 mm von der Öffnung 10 entfernt ein Bereich, der sich unter Druckbeaufschlagung durch das Fluid 5 selbstständig nach Art einer Wulst 11 aufbläht und dabei eine zusätzliche Abdichtung dieser Applikation bewirkt.

Auf eine detaillierte Beschreibung der einzelnen Abbildungen der Figurenfolge 7 kann im Weiteren verzichtet werden, da es sich mit Ausnahme eines kissenförmigen Applikators im Wesentlichen um Adaptionen der Figuren 2a bis 6 unter Zerteilung eines Ganzkörperanzugs in Einzelelemente handelt, die mindestens vom hygienischen Standpunkt her betrachtet unproblematischer in Handhabung und Wartung bzw. Reinigung sind.

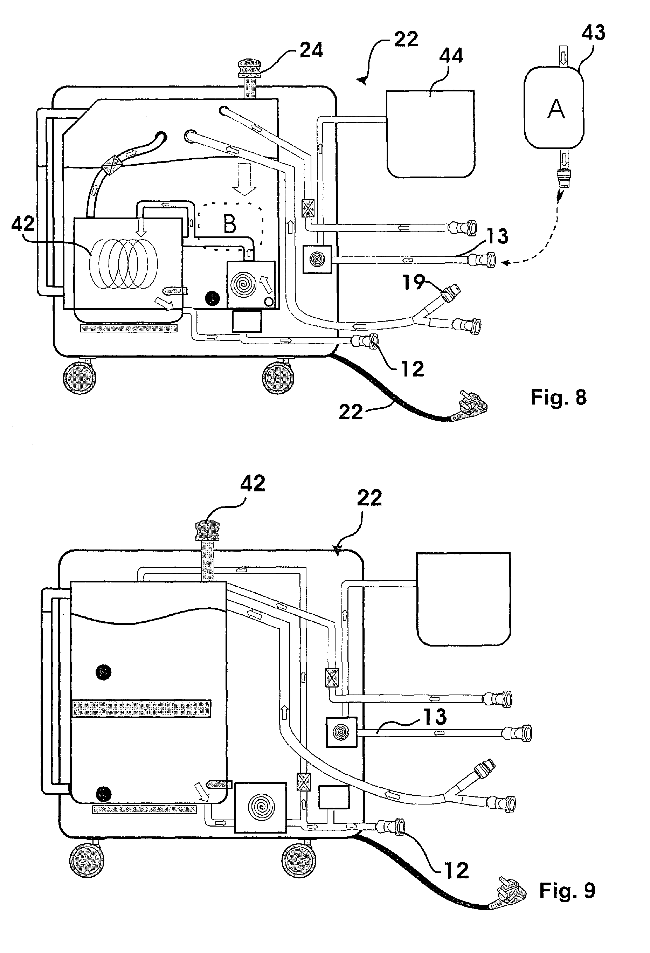

Besondere Vorteile von Ausführungsbeispielen von Versorgungseinheiten 22 werden nachfolgend unter Bezug auf die Abbildungen der Figuren 8 und 9 angegeben:

Figur 8 zeigt eine Versorgungseinheiten 22, bei der auf dem Einfüllstutzen 24 eines Hauptbehälters 39 ein Luftfilter 41 angeordnet ist, der nur dann kurz abgenommen wird, um Zusatzstoffe für das Fluid 5 einzufüllen. Diese Vorgehen verhindert das Eindringen von Viren und Bakterien, die sich in der Luft befinden und beim Abpumpen in die Versorgungseinheit 22 gesogen werden. Das Gerät hat in einem Ausgleichsbehälter einen Silberdraht 42, damit das stehende Fluid entkeimt wird. Es bleiben aufgrund der baulichen Anordnung immer etwa 15 Liter Fluid im System. Der Ablauf ist dann wie folgt: Fluid einfüllen (je nach Applikator ca. 5-20 Liter), Aufheizen auf 32°-39°C und die Behandlung mit dem angeschlossenen Applikator beginnen. Das Wasser wird über einen im Zulauf montierten Filter 43 zum Applikator 1 geleitet und somit werden Bakterien, Viren und Verunreinigungen vor jedem Patientenkontakt entfernt. Das Fluid aus dem Applikator wird nach ca. 20 minütiger Behandlung in einen separaten Behälter 44 gepumpt, um die eventuellen Keime vom Patienten nicht über den Rücklauf in das Gerät zu spülen. Dann wird das Gerät 22 und der Applikator 1 in einem 8 minütigen Spülgang gesäubert. Diese vergleichsweise langen Spülzeiten werden benötigt, da ja 15 Liter umgewälzt werden müssen. Auch der Applikator wird vorher chemisch gereinigt.

Die Einheit von Figur 9 stellt eine wesentliche Modifikation dar: Bei einem Desinfektionsgang werden 15 Liter Wasser vor Inbetriebnahme des Gesamtsystems aus Versorgungseinheit 22 und einem jeweiligen Applikator 1 in die Versorgungseinheit 22 gefüllt und auf 76°C erhitzt. Dann wird das heiße Wasser durch die Zulauf-, und Ablaufschläuche 12, 13 in den Applikator 1 und von dort als verbrauchte Reinigungsflüssigkeit in einen Ausguss gepumpt, wobei normales Wasser verwendet wird. Danach wird Fluid einfüllen, je nach Applikator ca. 5-20 Liter, und die Behandlung mit dem jetzt desinfizierten Applikator 1 beginnt. Das Fluid aus dem Applikator 1 wird nach ca. 20 minütiger Behandlung in einen separaten Behälter gepumpt, um die vom Patienten stammenden Keime und Verunreinigungen nicht über den Rücklauf in Versorgungseinheit 22 zu spülen. Dann wird die Versorgungseinheit 22 mit dem Applikator 1 in einem 2 minütigen Spülgang gesäubert, wobei sich die vergleichsweise kürzere Spülzeiten aus der Tatsache ergibt, dass das System nur noch 1-1,5 Liter Flüssigkeit in den Schlauchleitungen 12, 13 stehen hat. Ein wesentlicher Vorteil dieser Konstruktion besteht im Wesentlichen in der Möglichkeit des Einsatzes jeglicher gewünschten Beimischung im Fluid, da diese nicht durch Filter etc. beeinträchtigt werden. Der Applikator 1 muss zudem nicht extra chemisch gereinigt werden.

Mithin stehen dem vorstehend in Ausführungsbeispielen beschriebenen System Einsatz- und Anwendungsmöglichkeiten in den folgenden medizinischen Sektoren offen:
1. Allgemeine Schmerztherapie
2. Spezielle Schmerztherapie in der Rheumatologie
3. Dermatologie
4. Angiologie bzw. Gefäßerkrankungen
5. Wundheilung
6. Wellness/Anti-Aging Medizin
7. Physikalische Medizin

Ferner besteht ein wesentlicher Anwendungsfeld im Bereich der Kosmetik

Mit einer erfindungsgemäßen Vorrichtung steht eine neue Möglichkeit in der Therapie zur Verfügung. Dessen vielfältige Anwendungen begründen sich in folgenden Geräteeigenschaften:
a) Entspannung durch Wärme zwischen 32-39° C, die frei und individuell wählbar ist
b) Geringer Kontakt- und Auflagedruck
c) Pulseinrichtung für die kontaktlose Massage
d) Einschleusen von Medikamenten und Mineralien und Vitalstoffen in die oberen Hautschichten

Eine erfindungsgemäße Vorrichtung ermöglicht spezielle, neuartige und schonende Anwendungen. Nicht nur durch die regulierenden und wählbaren Temperaturbereiche von 32 - 39° C, sondern auch durch die Möglichkeiten der Einschleusung von Mineralien, Vitaminen und Pharmazeutika in die Haut und den Organismus. Dabei wird ein sanfter lokaler Massageeffekt ausgeübt. Die freie Wählbarkeit der Applikationen ermöglicht schonende Maßnahmen bei Schmerzpatienten durch eine individuelle Einstellung im speziell wählbaren Temperaturbereich von 32 - 39° C und der frei justierbaren Pulseinrichtung. Dadurch kann die geeignete und angenehm empfundene Erwärmung des Patienten mit dem Ziel der körperlichen und muskulären Entspannung erreicht werden. Jedoch auch eine Erfrischung und Schmerzlinderung durch Kühlung. Somit lassen sich die Anwendungen zu jeder Jahreszeit durchführen.

Der Kontaktdruck der hautanliegenden Anwendungsteile kann stufenlos in eine für den Patienten nahezu schmerzfreie Berührung mit dessen Haut gebracht werden. Dies ist ein wichtiges Kriterium in der Behandlung von Weichteilrheumatikern (Fibromyagie-Patienten) und auch Schmerzpatienten, die häufig sehr druckempfindlich bei entsprechenden herkömmlichen Massagen sind. Die speziellen Applikatoren lassen Anwendungen für jede Körperregion zu. Die häufig muskulären Verspannungen der Wirbelsäule aber auch der Extremmuskulatur erlauben mit einer erfindungsgemäßen Vorrichtung somit eine sanfte Lockerung und eröffnen das Feld der Applikationen bis in die physikalische Medizin.

Durch eine spezielle Pulseinrichtung ist es möglich eine kontaktlose und entspannende Massage durchzuführen. Dadurch wird ein weiterer Effekt und eine weitere Dimension in der Behandlung eröffnet. Der Pulser versetzt die durchströmende Flüssigkeit in eine regelbare Schwingung im unteren Hz-Bereich und ermöglicht somit eine unbelastende, aber effiziente Lockerungsbehandlung des Patienten. Dass sich dieser Effekt vielfältigst von der allgemeinen Schmerztherapie bis hin zur Entspannung im Wellness-Bereich anwenden lässt, liegt somit unmittelbar auf der Hand. Auch führt die Pulseinrichtung zu einer vermehrten Durchblutung. Damit lassen sich die weiteren Anwendungen im Bereich der schwer abheilenden Wunden und der Durchblutungsförderung in der Angiologie ableiten. Die periphere Durchblutung wird mit einer erfindungsgemäßen Vorrichtung mittels Temperatur und Pulsung angeregt. Dies kann zu einer Verbesserung der Wundheilung, gerade beim häufigen Ulcus cruris, führen. Durch das Zufügen von individuell frei wählbaren orthomolekularen und pharmazeutischen Substanzen und Essenzen können zusätzliche Effekte erzielt werden. Gerade in der Dermatologie können solche Anwendungen mit den Applikatoren durchgeführt werden, die eine Hautbehandlung von Körperteilen oder dem gesamten Körper möglich machen. Damit ergeben sich weitere Anwendungen wiederum in den anderen Anwendungsbereichen einer erfindungsgemäßen Vorrichtung. Von der Schmerztherapie über eine vermehrte Gewebedurchblutung bis hin zur Wellness- und Anti-Aging Medizin. Hier sollten spezielle Nährstoffe in besonderen Darreichungen in die Haut eingeschleust werden. Damit ist eine ganzheitliche Behandlung möglich.

Der Trägerflüssigkeit können Pharmazeutika und Vitalstoffe zugeführt werden. Als Trägerflüssigkeiten können neben Leitungswasser auch Mineralwasser und Salzkonzentrationen gewählt werden. Als mögliche Zusätze könnten Genestin, Sojaessenzen, Zink, Selen, antifaltenwirkende Substanzen, wie aber auch speziell zubereitetes Vitamin C und rückfettende Lipide in mikronisierter Form zur Anwendung kommen. Die genannten Substanzen stellen eine kleine Auswahl von Möglichkeiten dar. So leitet sich eine schonende Applikation vom Mineralwasser bis hin zu speziellen Zubereitungen ab. Neue Möglichkeiten ergeben sich so in allen vorstehend in den Punkten 1-6 genannten Bereichen der Anwendungen. Dass diese Effekte auch im Bereich der Wellness und Anti-Aging-Medizin einsetzbar sind, lässt sich aus dem Gesagten ableiten.

Durch einen Überdruck der strömenden Flüssigkeit, der vorzugsweise im Millibarbereich liegt, werden Applikationen in die Epidermis und in die obere Lederhaut eingebracht. Im Verlauf der Anwendung mit einer erfindungsgemäßen Vorrichtung, lassen sich Anwendungen vor, während und nach der Anwendung gestalten, so dass auch zu den genannten Zeitpunkten Hautcremes und spezielle Zubereitungen auf die Haut aufgetragen werden können.
Die Anwendungszeit mit einer erfindungsgemäßen Vorrichtung beträgt insgesamt etwa 10-15 Minuten, wobei auch eine Intervalltherapie mit Ruhepausen möglich ist. Gerade diese Ruhepausen ermöglichen es, gewählte und verfügbare Substrate einzuschleusen und einwirken zu lassen.

Bei Ausgestaltung des Umfeldes können lichttherapeutische Effekte, wie auch Düfte noch zusätzlich zur Anwendung kommen, damit die Effekte der Entspannung noch weiter intensiviert werden. In allen Bereichen können solche Effekte das Wohlfühlen und Wohlempfinden der Anwendungen mit einer erfindungsgemäßen Vorrichtung erhöht werden.

Neben den medizinischen Möglichkeiten einer erfindungsgemäßen Vorrichtung besitzt das System auch interessante wirtschaftliche Eigenschaften für den Käufer. Fünf wirtschaftliche Eigenschaften runden den positiven Eindruck des vorgestellten Systems ab:
1. Insgesamt bietet eine erfindungsgemäße Vorrichtung mit ihren Möglichkeiten einen sparsamen Umgang mit Wasser als Trägerflüssigkeit von maximal 70 Liter für eine Ganzkörperbehandlung. Da durch das geschlossene System kein Verdunstungseffekt entstehen kann, leistet eine erfindungsgemäße Vorrichtung auch mit den medizinischen Applikationen einen sparsamen Umgang. Ebenso treten keine Verunreinigungen der Trägerflüssigkeit auf, so dass mehrere Patienten mit der gleichen Trägerflüssigkeit behandelt werden können, wie durch ein entsprechendes Zertifikat des Paul-Ehrlich-Institutes nachgewiesen wird.
2. Spezielle Nasszellen sind nicht notwendig, so dass die Anwendungen in Räumlichkeiten mit angenehmer Atmosphäre durchgeführt werden können. Lediglich ein Wasseranschluss in der Nähe zum Befüllen einer erfindungsgemäßen Vorrichtung und eine Möglichkeit zum Entleeren des Behälters und Spülen der Applikatoren sind notwendig.
3. Eine erfindungsgemäße Vorrichtung ermöglicht Effekte in sehr kurzen Zeiteinheiten.
4. Eine erfindungsgemäße Vorrichtung ist einfach und ohne Fachpersonal bedienbar.
5. Kurze Amortisationszeiten für Ärzte, Kliniken und Anwender resultieren aus diesen Vorteilen.

### Bezugszeichenliste

- 1: Vorrichtung
- 2: menschlicher Körper
- 3: Fluid-undurchlässige Schicht
- 4: mindestens teilweise Fluid-durchlässige Schicht
- 5: Fluid
- 5a: ausgetretenes Fluid im Kontakt mit der Körperoberfläche
- 6: Körperoberfläche
- 7: Öffnung zum Fluidaustritt
- 8: dünne Schicht von Fluid
- 9 10: Öffnung
- 11: Wulst / Abdichtung
- 12: Zulauf (-Schlauch)
- 13: Ablauf (-Schlauch)
- 14 15: Stützkörper
- 16: Stützpolster
- 17: Reißverschluss
- 18: Anzugende
- 19: Anzugende
- 20: Schnellkupplung
- 21 22: Versorgungseinheit
- 23: Druckbehälter
- 24: Einfüllstutzen
- 25: Frequenzeinheit
- 26: Pulser
- 27: Temperaturregeleinheit
- 28: Temperaturfühler
- 29: Aufwärmeinheit
- 30: Druckregeleinheit
- 31: Drucksensor
- 32: Ventil
- 33: Druckpumpe
- 34: Versorgungsanschluss
- 35: Entlüftungsventil
- 36: Aufhänglasche
- 37 38: punktförmige Verbindung
- 39: Hauptbehälter
- 40: Absaugpumpe
- 41: Luftfilter
- 42: Silberdraht
- 43: Filter
- 44 45: Behälter

- a: Fußbereich
- b: Beinbereich
- c: Arm
- d: Rücken- und Brustbereich
- e: Beinbereich
- f: Fußbereich
- g: linke Anzugseite
- h: rechte Anzugseite

## Patentansprüche

1. Verfahren zur äußerlichen Fluid-Behandlung eines menschlichen und/oder tierischen Körpers (2), bei dem ein zu behandelnder Bereich der Körperoberfläche (6) mit einer undurchlässigen Schicht (3) abgedeckt und
ein Fluid (5) unter dieser Schicht (3) so eingeleitet wird, dass es sich in Kontakt mit der Körperoberfläche (6) befindet,
**dadurch gekennzeichnet,**
**dass** eine zusätzliche, in Randbereichen mit der undurchlässigen Schicht (3) verbundene, mindestens teilweise Fluid- durchlässige Schicht (4) zwischen der undurchlässigen Schicht (3) und der Körperoberfläche (6) angeordnet wird und
das Fluid (5) über einen Zulauf (12) derart zwischen diesen Schichten (3, 4) eingeleitet wird, dass die mindestens teilweise Fluid-durchlässige Schicht (4) an die Körperoberfläche (6) angelegt wird und
eine geringe Menge an Fluid (5a) durch Öffnungen (7) dieser mindestens teilweise Fluid-durchlässigen Schicht (4) hindurch auf einen Teil des abgedeckten Bereichs der Körperoberfläche (6) austritt.

2. Verfahren nach Anspruch 1,
**dadurch gekennzeichnet, dass** die mindestens teilweise Fluid-durchlässige Schicht (4) durch den Druck des Fluids (5) im Wesentlichen abdichtend an die Körperoberfläche (6) angedrückt wird.

3. Verfahren nach Anspruch 1 oder 2,
**dadurch gekennzeichnet, dass** eine Temperierung während des Betriebs durch Wärmestrahlung von außen her oder durch eine körpernah angeordnete Heizfolie vorgenommen wird, die vorzugsweise ein Bestandteil der aus den beiden Schichten (3, 4) bestehenden Abdeckung ist.

4. Verfahren nach einem oder mehreren der vorhergehenden Ansprüche, **dadurch gekennzeichnet, dass** durch pulsende Beaufschlagung des Fluids (5) eine aktive mechanische Massage durchgeführt wird, wobei die Pulsung insbesondere über den Zulauf (12) auf das Fluid (5) zwischen den Schichten (3, 4) übertragen wird.

5. Verfahren nach einem oder mehreren der vorhergehenden Ansprüche, **dadurch gekennzeichnet, dass** durch unter pulsender Beaufschlagung des Fluids (5) in verschiedenen Kammern nach Art einer Lymphmassage durchgeführt wird.

6. Verfahren nach einem oder mehreren der vorhergehenden Ansprüche, **dadurch gekennzeichnet, dass** die Vorrichtung durch das Fluid (5) zwischen der mindestens teilweise Fluid- durchlässige Schicht (4) und der undurchlässigen Schicht (3) im Wesentlichen frei durchströmt wird, so dass eine Temperatureinstellung und ein Innendruck über einen Zu- und Abstrom von Fluid (5) und dessen Zuflusstemperatur eingeregelt wird.

7. Verfahren nach einem oder mehreren der vorhergehenden Ansprüche, **dadurch gekennzeichnet, dass** dem Fluid (5) ätherische Öle, hautheilende Substanzen, Mineralstoffe, Salze, Vitamine, Sauerstoff- oder ein sonstiges Gas oder Gasgemisch zugesetzt werden.

8. Verfahren nach einem oder mehreren der vorhergehenden Ansprüche, **dadurch gekennzeichnet, dass** die zwei Schichten (3, 4) umfassende Abdeckung über den Zulauf (12) durch eine Versorgungseinheit (22) mit Fluid (5) versorgt werden, das in der Versorgungseinheit (22) hinsichtlich Temperatur, Druck, Pulsung, Zusammensetzung und/oder Beimischungen regelbar und/oder einstellbar sowie in bakteriologischer und hygienischer Hinsicht durch Filterung, Bestrahlung, oligodynamische und/oder thermische Prozesse u.a. aufbereitet wird.

9. Vorrichtung zur äußerlichen Fluid-Behandlung eines menschlichen und/oder tierischen Körpers (2), bei der über einen zu behandelnden Bereich der Körperoberfläche (6) eine Fluid-undurchlässigen Schicht (3) angeordnet und ein Zulauf (12) zur Einspeisung eines Fluids (5) unter dieser Schicht (3) und in oberflächlichem Kontakt mit der Körperoberfläche (6) vorgesehen ist,
**dadurch gekennzeichnet,**
**dass** eine mindestens teilweise Fluid-durchlässige Schicht (4) zwischen der undurchlässigen Schicht (3) und der Körperoberfläche (6) angeordnet ist,
die Schichten (3, 4) miteinander zu einer Einheit verbunden sind, insbesondere in einem Randbereich,
so dass das Fluid (5) zwischen diesen Schichten (3, 4) derart einfüllbar ist, dass die mindestens teilweise Fluid- durchlässige Schicht (4) an den Körper (2) angelegt ist.

10. Vorrichtung nach Anspruch 9,
**dadurch gekennzeichnet, dass** die Vorrichtung (1) als im Wesentlichen kissenförmige Kompresse ausgebildet, in und/oder an der mindestens eine Bandage oder eine Schnur zur Fixierung über einem zu behandelnden Bereich der Körperoberfläche (6) vorgesehen ist.

11. Vorrichtung nach einem der vorhergehenden Ansprüche 9 oder 10, **dadurch gekennzeichnet, dass** die Vorrichtung (1) mindestens eine Öffnung (10) zum Umschließen eines Abschnitts eines zu behandelnden Körpers (2) aufweist und als Schuh, Arm-, Bein-, Torso-Manschette oder als Ganzkörperanzug ausgebildet ist, wobei die Vorrichtung (1) als doppelwandiger Ganzkörperanzug in ein- oder mehrteiliger Ausgestaltung ausgebildet ist, der einen Körper (2) mit Füßen und Händen bis zum Hals umschließt, vorzugsweise jedoch den Bereich der Achseln und/oder der Genitalien ausspart.

12. Vorrichtung nach Anspruch 11, **dadurch gekennzeichnet,**
**dass** die Öffnung (10) durch eine ringförmige Wulst (11) umfasst ist, die aus dem Material der Fluid- undurchlässigen Schicht (3) besteht und derart ausgebildet ist, dass sie bei Beaufschlagung der Vorrichtung (1) mit Fluid (5) als zusätzliche Abdichtung an die Körperoberfläche (6) angelegt wird.

13. Vorrichtung nach einem der vorhergehenden Ansprüche 9 bis 12, **dadurch gekennzeichnet, dass** die Vorrichtung (1) neben einem Zulauf (12) auch einen Ablauf (13) aufweist.

14. Vorrichtung nach einem der vorhergehenden Ansprüche 9 bis 13, **dadurch gekennzeichnet, dass** in der Vorrichtung (1) eine Heizfolie vorgesehen ist.

15. Vorrichtung nach einem der vorhergehenden Ansprüche 9 bis 14, **dadurch gekennzeichnet, dass** zwischen den Schichten (3, 4) Stützkörper (15) vorgesehen sind, die insbesondere als ungefähr I-förmige Körper aus einem elastischen Material ausgebildet sind und eine Art von Stützpolster (16) in Bereichen bilden, um die Ausbildung von Stauungen des Fluids (5) zwischen den Schichten (3, 4) und/oder strömungstoten Bereichen zu unterbinden.

16. Vorrichtung nach einem der vorhergehenden Ansprüche 9 bis 15, **dadurch gekennzeichnet, dass** die Vorrichtung (1) mit einer externen Versorgungseinheit (22) verbunden ist, in der Mittel zur Regelung und/oder Steuerung von Temperatur, Druck, Pulsung, Zusammensetzung und/oder Beimischungen des Fluids (5) sowie Analyse und/oder Überwachung hinsichtlich bakteriologischer und hygienischer Vorgaben des Fluids (5) und/oder der gesamten Anordnung vorgesehen sind.

17. Vorrichtung nach einem der vorhergehenden Ansprüche 9 bis 16, **dadurch gekennzeichnet, dass** in der Vorrichtung (1) zwischen den Schichten (3, 4) mehrere miteinander verbundene Kammern als Systeme mit vorgegebenen und/oder voneinander trennbaren oder getrennt ansteuerbaren Wasserkreisläufen vorgesehen sind.

18. Vorrichtung nach einem der vorhergehenden Ansprüche 9 bis 16, **dadurch gekennzeichnet, dass** die Schichten (3, 4) durch im Wesentlichen kreisförmige Verbindungen oder Verschweißungen als im Wesentlichen punktförmige Verbindungen (38), die voneinander beabstandet über die Schichten (3, 4) verteilt sind, verbunden sind, insbesondere in regelmäßiger Anordnung nach Art einer Steppdecke.

19. Vorrichtung nach einem der vorhergehenden Ansprüche 9 bis 18, **dadurch gekennzeichnet, dass** zur Versorgung der Vorrichtung (1) mit Fluid (5) Zulauf- und Rücklaufschläuche (12, 13) mit Durchmesserunterschieden zum Druckaufbau des Fluids (5) zwischen den Schichten (3, 4) der Vorrichtung (1) vorgesehen sind.

20. Verwendung einer Vorrichtung nach einem oder mehreren der Ansprüche 9 bis 19 und/oder Anwendung eines Verfahrens nach einem oder mehreren der Ansprüche 1 bis 8 im Rettungsbereich, insbesondere zur Behandlung von Unterkühlungen oder Verbrennungen.

## Geänderte Patentansprüche

### Geänderte Patentansprüche gemäss Regel 86(2) EPÜ.

**1.** Verfahren zur äußerlichen Fluid-Behandlung eines menschlichen und/oder tierischen Körpers (2) im Beauty- sowie Wellness-Bereich, bei dem ein zu behandelnder Bereich der Körperoberfläche (6) mit einer undurchlässigen Schicht (3) abgedeckt und
ein Fluid (5) unter dieser Schicht (3) so eingeleitet wird, dass es sich in Kontakt mit der Körperoberfläche (6) befindet,
**dadurch gekennzeichnet,**
**dass** eine zusätzliche, in Randbereichen mit der undurchlässigen Schicht (3) verbundene, mindestens teilweise Fluid- durchlässige Schicht (4) zwischen der undurchlässigen Schicht (3) und der Körperoberfläche (6) angeordnet wird und
das Fluid (5) über einen Zulauf (12) derart zwischen diesen Schichten (3, 4) eingeleitet wird, dass die mindestens teilweise Fluid-durchlässige Schicht (4) an die Körperoberfläche (6) angelegt wird und
eine geringe Menge an Fluid (5a) durch Öffnungen (7) dieser mindestens teilweise Fluid-durchlässigen Schicht (4) hindurch auf einen Teil des abgedeckten Bereichs der Körperoberfläche (6) austritt.

**2.** Verfahren nach Anspruch 1,
**dadurch gekennzeichnet, dass** die mindestens teilweise Fluid-durchlässige Schicht (4) durch den Druck des Fluids (5) im Wesentlichen abdichtend an die Körperoberfläche (6) angedrückt wird.

**3.** Verfahren nach Anspruch 1 oder 2,
**dadurch gekennzeichnet, dass** eine Temperierung während des Betriebs durch Wärmestrahlung von außen her oder durch eine körpernah angeordnete Heizfolie vorgenommen wird, die vorzugsweise ein Bestandteil der aus den beiden Schichten (3, 4) bestehenden Abdeckung ist.

**4.** Verfahren nach einem oder mehreren der vorhergehenden Ansprüche, **dadurch gekennzeichnet, dass** durch pulsende Beaufschlagung des Fluids (5) eine aktive mechanische Massage durchgeführt wird, wobei die Pulsung insbesondere über den Zulauf (12) auf das Fluid (5) zwischen den Schichten (3, 4) übertragen wird.

**5.** Verfahren nach einem oder mehreren der vorhergehenden Ansprüche, **dadurch gekennzeichnet, dass** durch unter pulsender Beaufschlagung des Fluids (5) in verschiedenen Kammern nach Art einer Lymphmassage durchgeführt wird.

**6.** Verfahren nach einem oder mehreren der vorhergehenden Ansprüche, **dadurch gekennzeichnet, dass** die Vorrichtung durch das Fluid (5) zwischen der mindestens teilweise Fluid- durchlässige Schicht (4) und der undurchlässigen Schicht (3) im Wesentlichen frei durchströmt wird, so dass eine Temperatureinstellung und ein Innendruck über einen Zu- und Abstrom von Fluid (5) und dessen Zuflusstemperatur eingeregelt wird.

**7.** Verfahren nach einem oder mehreren der vorhergehenden Ansprüche, **dadurch gekennzeichnet, dass** dem Fluid (5) ätherische Öle, hautheilende Substanzen, Mineralstoffe, Salze, Vitamine, Sauerstoff- oder ein sonstiges Gas oder Gasgemisch zugesetzt werden.

**8.** Verfahren nach einem oder mehreren der vorhergehenden Ansprüche, **dadurch gekennzeichnet, dass** die zwei Schichten (3, 4) umfassende Abdeckung über den Zulauf (12) durch eine Versorgungseinheit (22) mit Fluid (5) versorgt werden, das in der Versorgungseinheit (22) hinsichtlich Temperatur, Druck, Pulsung, Zusammensetzung und/oder Beimischungen regelbar und/oder einstellbar sowie in bakteriologischer und hygienischer Hinsicht durch Filterung, Bestrahlung, oligodynamische und/oder thermische Prozesse u.a. aufbereitet wird.

**9.** Vorrichtung zur äußerlichen Fluid-Behandlung eines menschlichen und/oder tierischen Körpers (2), bei der über einen zu behandelnden Bereich der Körperoberfläche (6) eine Fluid-undurchlässigen Schicht (3) angeordnet und ein Zulauf (12) zur Einspeisung eines Fluids (5) unter dieser Schicht (3) und in oberflächlichem Kontakt mit der Körperoberfläche (6) vorgesehen ist,
**dadurch gekennzeichnet,**
**dass** eine mindestens teilweise Fluid-durchlässige Schicht (4) zwischen der undurchlässigen Schicht (3) und der Körperoberfläche (6) angeordnet ist,
die Schichten (3, 4) miteinander zu einer Einheit verbunden sind, insbesondere in einem Randbereich,
so dass das Fluid (5) zwischen diesen Schichten (3, 4) derart einfüllbar ist, dass die mindestens teilweise Fluid- durchlässige Schicht (4) an den Körper (2) angelegt ist.

**10.** Vorrichtung nach Anspruch 9,
**dadurch gekennzeichnet, dass** die Vorrichtung (1) als im Wesentlichen kissenförmige Kompresse ausgebildet, in und/oder an der mindestens eine Bandage oder eine Schnur zur Fixierung über einem zu behandelnden Bereich der Körperoberfläche (6) vorgesehen ist.

**11.** Vorrichtung nach einem der vorhergehenden Ansprüche 9 oder 10, **dadurch gekennzeichnet, dass** die Vorrichtung (1) mindestens eine Öffnung (10) zum Umschließen eines Abschnitts eines zu behandelnden Körpers (2) aufweist und als Schuh, Arm-, Bein-, Torso-Manschette oder als Ganzkörperanzug ausgebildet ist, wobei die Vorrichtung (1) als doppelwandiger Ganzkörperanzug in ein- oder mehrteiliger Ausgestaltung ausgebildet ist, der einen Körper (2) mit Füßen und Händen bis zum Hals umschließt, vorzugsweise jedoch den Bereich der Achseln und/oder der Genitalien ausspart.

**12.** Vorrichtung nach Anspruch 11, **dadurch gekennzeichnet, dass** die Öffnung (10) durch eine ringförmige Wulst (11) umfasst ist, die aus dem Material der Fluid- undurchlässigen Schicht (3) besteht und derart ausgebildet ist, dass sie bei Beaufschlagung der Vorrichtung (1) mit Fluid (5) als zusätzliche Abdichtung an die Körperoberfläche (6) angelegt wird.

**13.** Vorrichtung nach einem der vorhergehenden Ansprüche 9 bis 12, **dadurch gekennzeichnet, dass** die Vorrichtung (1) neben einem Zulauf (12) auch einen Ablauf (13) aufweist.

**14.** Vorrichtung nach einem der vorhergehenden Ansprüche 9 bis 13, **dadurch gekennzeichnet, dass** in der Vorrichtung (1) eine Heizfolie vorgesehen ist.

**15.** Vorrichtung nach einem der vorhergehenden Ansprüche 9 bis 14, **dadurch gekennzeichnet, dass** zwischen den Schichten (3, 4) Stützkörper (15) vorgesehen sind, die insbesondere als ungefähr I-förmige Körper aus einem elastischen Material ausgebildet sind und eine Art von Stützpolster (16) in Bereichen bilden, um die Ausbildung von Stauungen des Fluids (5) zwischen den Schichten (3, 4) und/oder strömungstoten Bereichen zu unterbinden.

**16.** Vorrichtung nach einem der vorhergehenden Ansprüche 9 bis 15, **dadurch gekennzeichnet, dass** die Vorrichtung (1) mit einer externen Versorgungseinheit (22) verbunden ist, in der Mittel zur Regelung und/oder Steuerung von Temperatur, Druck, Pulsung, Zusammensetzung und/oder Beimischungen des Fluids (5) sowie Analyse und/oder Überwachung hinsichtlich bakteriologischer und hygienischer Vorgaben des Fluids (5) und/oder der gesamten Anordnung vorgesehen sind..

**17.** Vorrichtung nach einem der vorhergehenden Ansprüche 9 bis 16, **dadurch gekennzeichnet, dass** in der Vorrichtung (1) zwischen den Schichten (3, 4) mehrere miteinander verbundene Kammern als Systeme mit vorgegebenen und/oder voneinander trennbaren oder getrennt ansteuerbaren Wasserkreisläufen vorgesehen sind.

**18.** Vorrichtung nach einem der vorhergehenden Ansprüche 9 bis 16, **dadurch gekennzeichnet, dass** die Schichten (3, 4) durch im Wesentlichen kreisförmige Verbindungen oder Verschweißungen als im Wesentlichen punktförmige Verbindungen (38), die voneinander beabstandet über die Schichten (3, 4) verteilt sind, verbunden sind, insbesondere in regelmäßiger Anordnung nach Art einer Steppdecke.

**19.** Vorrichtung nach einem der vorhergehenden Ansprüche 9 bis 18, **dadurch gekennzeichnet, dass** zur Versorgung der Vorrichtung (1) mit Fluid (5) Zulauf- und Rücklaufschläuche (12, 13) mit Durchmesserunterschieden zum Druckaufbau des Fluids (5) zwischen den Schichten (3, 4) der Vorrichtung (1) vorgesehen sind.
